# FASCICULE DE BREVET EUROPEEN

(11) **EP 0 889 885 B1**
(45) Date de publication et mention de la délivrance du brevet: **29.05.2002**
(21) Numéro de dépôt: 96944097.3
(22) Date de dépôt: 27.12.1996
(51) Int. Cl.: C07D 305/14, C07F 7/18

(54) **PROCEDE DE PROTECTION SELECTIVE DES DERIVES DE LA BACCATINE ET SON UTILISATION DANS LA SYNTHESE DES TAXANES**
VERFAHREN ZUM AUSGEWÄHLTEM SCHUTZ VON BACCATINDERIVATEN UND IHRE VERWENDUNG FÜR DIE SYNTHESE VON TAXANEN
METHOD FOR SELECTIVE PROTECTION OF BACCATIN DERIVATIVES AND ITS APPLICATION TO TAXANE SYNTHESIS

(30) Priorité: 27.12.1995 FR 9515557
(43) Date de publication de la demande: 13.01.1999
(73) Titulaire: Societé d'Etude et de Recherche en Ingenierie Pharmaceutique Seripharm, 72000 Le Mans (FR)
(72) Inventeur: CHANTELOUP, Luc, F-72190 Sargé-lès-Le Mans (FR); CHAUVEAU, Bruno, 94, rue de Sargé F-72000 Le Mans (FR); CORBIN, Christine, F-72700 Etival-lès-Le Mans (FR); DHAL, Robert, Pruillé-le-Chétif F-72700 Le Mans (FR); LE GUEN, Sonia, F-72000 Le Mans (FR); LAMY, Arnaud, F-72650 La Milesse (FR); LEZE, Antoine, F-72000 Le Mans (FR); ROBIN, Jean-Pierre, F-72000 Le Mans (FR)
(74) Mandataire: Le Pennec, Magali
(86) Numéro de dépôt international: FR9602097
(87) Numéro de publication internationale: WO9724345

(56) Documents cités:
- EP-A- 0 253 739
- WO-A-95/20582

## Description

La présente invention concerne de nouveaux intermédiaires pour l'hémisynthèse de taxanes et leurs procédés de préparation.

Les taxanes, substances naturelles dont le squelette diterpènique est généralement estérifié par une chaîne latérale β-aminoacide dérivée de la N-alcoyl- ou N-aroyl-phényl-isosérine, sont connus comme agents anticancéreux. On compte plusieurs dizaines de taxanes isolés à partir de Taxacées du genre Taxus, comme par exemple, le PACLITAXEL (R1 = Ac, R2 = Ph, R3 = R4 = H), la céphalomanine, leurs dérivés désacétylés en position 10, ou les baccatines (dérivés sans chaîne latérale) représentés par les formules 1 et 2 ci-dessous.

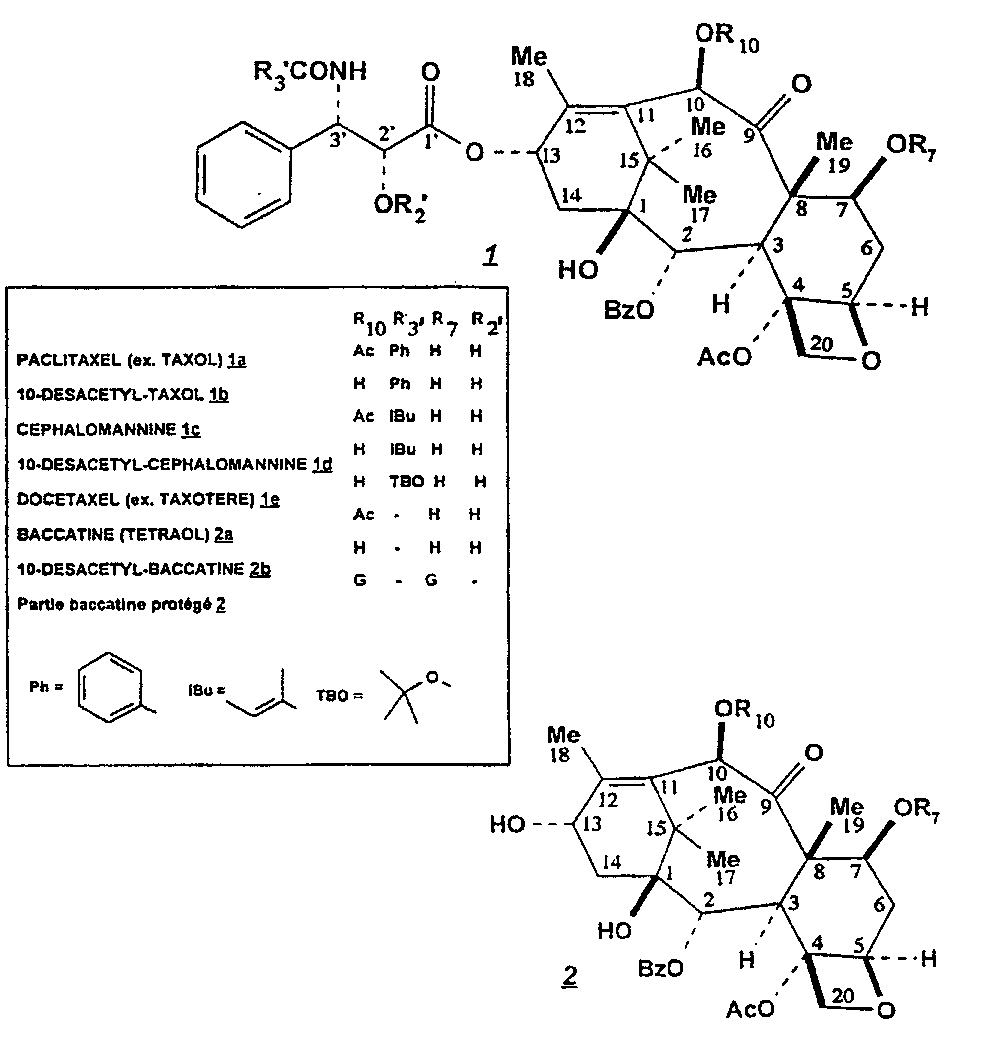

Dans le souci de ne pas épuiser rapidement sa source d'origine, T. brevifolia, des chercheurs français ont cherché à isoler le PACLITAXEL à partir de parties renouvelables (les feuilles) de l'if européen. Ils ont ainsi mis en évidence le précurseur biogénétique probable des taxanes, la 10-désacétylbaccatine III, tremplin de choix pour l'hémisynthèse, en raison de sa relative abondance dans les extraits de feuilles.

L'hémisynthèse des taxanes, comme le PACLITAXEL ou le DOCETAXEL (R1 = Ac, R2 = t.butyloxy, R3 = R4 = H), consiste donc à estérifier l'hydroxy en 13 d'un dérivé protégé de la baccatine ou de la 10-désacétylbaccatine III, avec un dérivé d'acide β-aminé.

Différents procédés d'hémisynthèse du PACLITAXEL ou du DOCETAXEL sont décrits dans l'état de la technique (EP-0 253 738, EP-0 336 840, EP-0 336 841, EP-0 495 718, WO 92/09589, WO 94/07877, WO 94/07878, WO 94/07879, WO 94/10169, WO 94/12482, EP-0 400 971, EP-0 428 376, WO 94/14787). Deux ouvrages récents [I. Georg, T.T. Chen, I Ojima, and D.M. Vyas, "Taxane Anticancer Agents, Basic Science and Current Status", ACS Symposium Series 583, Washington (1995)] et surtout [Matthew Suffness, "TAXOL® Science and Applications" CRC press (1995) et 1500 références citées] comprennent des compilations exhaustives des hémisynthèses de taxanes.

La monoprotection sélective de taxanes dihydroxylés en position 7 et 10 n'a pu jusqu'à présent être obtenue qu'à l'aide de groupements éthers trialkylsilanes (EP-A-0 336 840). Cette protection présente un grand intérêt en raison d'une part de la rareté par exemple de la baccatine III précurseur direct du PACLITAXEL et, d'autre part, de l'impossibilité de fabriquer celle-ci dans des conditions rentables à partir de son congénère beaucoup plus facilement disponible, la 10-désacétylbaccatine III, sans une protection sélective efficace en position 10.

En effet, l'usage de groupements courants comme des groupements acétates, ou même des haloalcoxycarbonyles faiblement encombrés tels le classique trichloroéthoxycarbonyle pour protéger des dihydroxytaxanes tels que la 10-désacétylbaccatine III, présente l'inconvénient de n'être aucunement sélectif. Par contre, ces derniers ont été jusqu'à présent largement utilisés dans la déprotection non sélective des 7,10-dihydroxytaxanes tels que la 10-désacétylbaccatine III, pour donner la 7,10-di-(2,2,2-trichloroéthoxycarbonyloxy)-10-désacétylbaccatine III, intermédiaire-clé dans la synthèse du DOCETAXEL (EP-A-0 336 841).

L'utilisation de trialkylsilanes pour la protection de l'hydroxyle situé en 7 de taxanes dihydroxylés en position 7 et 10, présente en raison de la relative labilité de ces groupements protecteurs vis-à-vis de milieux légèrement acides un certain nombre d'inconvénients importants venant contrebalancer leur bonne sélectivité.
1/ L'acylation ultérieure en 10, qui justifie la protection sélective préalable en 7, dont il va être question dans la présente invention, donne lieu à des rendements moyens 50 à 60 % en raison de la facilité des éthers trialkylsilylés à être substitués par le groupement acylant présent dans le milieu réactionnel, cette situation étant aggravée par la plus grande difficulté à estérifier la position 10 quand la position 7 est déjà occupée par un groupement encombrant. Cette réaction secondaire pourrait être évitée, à condition de la réaliser à très basse température en utilisant un alcoolate alcalin comme intermédiaire, mais introduirait un double inconvénient nouveau, pour une synthèse industrielle, comme indiqué dans la demande WOA-94/14787.
2/ La sensibilité des éthers alkylsilylés aux conditions acides limite les possibilités d'utiliser des réactifs acides dans les traitements de la séquence synthétique ultérieure, donc, rend beaucoup plus étroit le champ d'application de ces groupements protecteurs. De plus, il convient de remarquer que des homologues supérieurs tels que l'éther ter-butyldiméthylsilyle seraient plus résistants à ces conditions, mais leur mise en place est rendue impossible en raison de leur trop grand encombrement stérique.
3/ La sensibilité bien connue des éthers alkylsilylés aux solvants hydroxylés tels que l'eau et les alcools rend impossible l'usage de ces solvants dans les recristallisations, systèmes pourtant précieux car très purifiants dans cette série de composés, ce qui est un autre inconvénient important.
4/ La décomposition partielle, lors des purifications chromatographiques industrielles des intermédiaires porteurs d'éthers silylés, n'autorise pratiquement pas cette technique de purification qui est pourtant précieuse dans l'optique de l'obtention de matières premières pharmaceutiques à haute valeur ajoutée de haute pureté.

Les taxoïdes non porteurs de chaîne latérale de formule **3a** suivante : 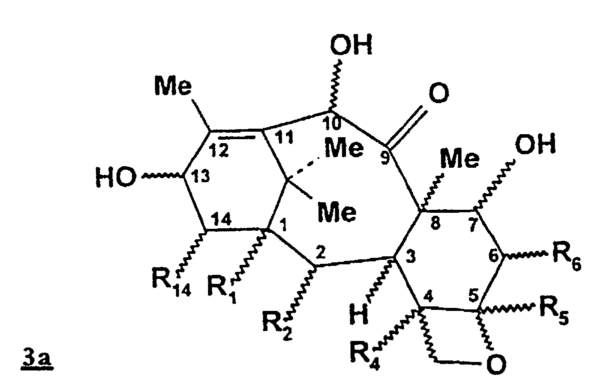 dans laquelle
R₁, R₂, R₄, R₅, R₆, R₁₄ représentent indépendamment l'un de l'autre un radical Q, avec :
Q = R, H, OH, OR, SH, SR, OCOR, OCOOR, HCO, X, et
X = halogène et,
R représente, indépendamment l'un de l'autre un radical alkyle linéaire ou ramifié, saturé ou insaturé, un radical perhalogénoalkyle, un radical hétéroalkyle linéaire ou ramifié, saturé ou insaturé, un radical cycloalkyle saturé ou insaturé, un radical hétérocycloalkyle saturé ou insaturé, un radical aryle, aralkyle, les radicaux pouvant être substitués, en particulier par un ou plusieurs halogènes ou non, leurs assemblages simples multiples combinés, et plus généralement toute combinaison les contenant sous forme de motifs simples ou répétés,
et en particulier les dérivés de formule générale 2 comme par exemple la 10-désacétylbaccatine III présentent souvent au moins quatre hydroxyles libres, l'un tertiaire, en position 1, très encombré et seulement estérifiable dans des conditions drastiques, les trois autres, secondaires, en position 7, 10 et 13, plus faciles à estérifier, que l'on peut, sur la base de leur encombrement stérique, diviser en deux groupes de réactivité :
- le premier, en position 13, est relativement peu réactif à l'estérification dans des conditions réactionnelles standards (y compris, selon la littérature, pour la mise en place de la chaîne latérale) ;
- le second groupe, en positions 7 et 10, qui intéresse l'objet de la présente invention, peut être divisé à son tour en deux niveaux subtilement différents de réactivités vis-à-vis de l'estérification ou de la carbonatation, légèrement en faveur de l'hydroxyle en position 7.

Il ressort également de nos travaux personnels que les deux sites d'hydroxylation présentent un cas d'interaction allostérique : la mise en place d'un ester encombré dans l'une des positions 7 ou 10, modifie la disponibilité stérique de l'autre position.

La présente invention concerne donc un nouveau procédé de protection sélective en position 7 des 7,10-dihydroxytaxanes de formule générale **3a** ci-dessus, par la mise en oeuvre de conditions réactionnelles particulières, simultanément à l'utilisation des groupements alcoxycarbonyles substitués en 2 (ou β) plus encombrés que le 2,2,2-trichloroéthoxycarbonyle (lequel reste non sélectif dans nos conditions réactionnelles particulières), capables d'être enlevés par un mécanisme d'élimination β (*Protecting Groups,* P.J. Kocienski, Thieme Verlag Ed., p. 7 (1994) et références citées), et ne présentant aucun des inconvénients des éthers trialkylsilylés mentionnés ci-dessus.

La demande de brevet WO-A-94/07877 décrit un procédé de synthèse de taxanes, pour lequel on peut employer le 2-trichlorométhyl-2-propoxycarbonyle comme groupement protecteur des hydroxyles 7 et 10 de la 10-désacétylbaccatine III. Il ressort toutefois de la description que ce groupement n'est pas employé pour la protection sélective de l'hydroxyle en 7, mais pour la protection des deux hydroxyles 7 et 10, nécessaire à la préparation du DOCETAXEL, seul produit réellement préparé dans les exemples, avec de surcroît le seul emploi exemplifié du 2,2,2-trichloroéthoxycarbonyle comme groupement protecteur. L'enseignement de cette demande de brevet tend donc à écarter l'homme du métier de l'usage de groupements β-halo-alcoxycarbonyles même plus encombrés que le 2,2,2-trichloroéthoxycarbonyle pour la préparation de la baccatine III protégée en 7, à partir de la 10-désacétylbaccatine III.

L'invention concerne également les nouveaux intermédiaires en résultant, le procédé d'acylation sélective en 10 (par rapport à l'hydroxyle situé en 13) des taxanes protégés en 7 et les nouveaux 7-(alcoxycarbonyloxy-β-substitués)-10-acyloxy-taxanes en résultant, leur utilisation dans le couplage avec des chaînes latérales de taxanes et les nouveaux intermédiaires taxanes protégés en résultant.

Enfin, la présente invention consiste à décrire l'enlèvement sélectif des groupements protecteurs β-substitués-alcoxycarbonyles aboutissant aux taxanes finaux, en particulier le DOCETAXEL.

La présente invention concerne donc tout d'abord en un procédé de préparation de dérivés 7-(2,2,2-trichloro-terbutoxycarbonyl)-10-hydroxy-taxanes répondant à la formule **3b** suivante : 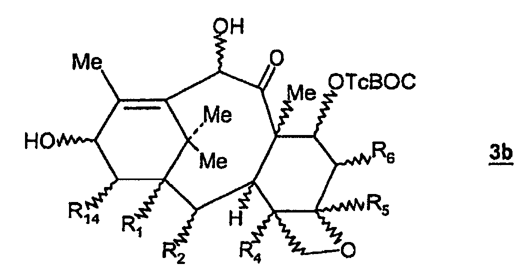 dans laquelle
R₁, R₂, R₄, R₅, R₆ et R₁₄ représentent indépendamment l'un de l'autre un radical Q, avec :
Q = H, OH, R, OR, SH, SR, OCOR, OCOOR, HCO, X, et
X = halogène,
R représente, indépendamment l'un de l'autre un radical alkyle ou alkylène linéaire ou ramifié, un radical perhalogénoalkyle, un radical hétéroalkyle ou hétéroalkylène linéaire ou ramifié, un radical cycloalkyle ou cycloalkylène, un radical hétérocycloalkyle ou hétérocycloalkylène, un radical aryle, aralkyle, les radicaux pouvant être substitués, en particulier par un ou plusieurs halogènes,
par addition lente à une température supérieure à la température ambiante, de préférence comprise entre 20 et 80°C, du chlorure de 2,2,2-trichloro-terbutoxycarbonyl dilué dans un solvant approprié, sur une solution fortement agitée du 7-10-dihydroxytaxane de formule générale **3a** 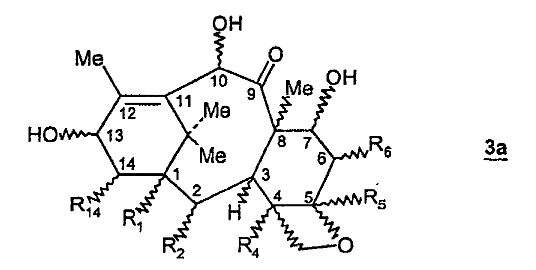 dans laquelle
R₁, R₂, R₄, R₅, R₆ et R₁₄ sont définis ci-dessus,
en présence de pyridine et/ou d'une pyridine substituée encombrée, comme par exemple la 4-pyrolidinopyridine ou la diméthyl-aminopyridine.

D'une manière avantageuse, l'estérification est conduite avec 1 à 1,5 équivalents de chloroformiate par rapport au 7-10-dihydroxytaxane de formule générale **3a**.

Les 7-(2,2,2-trichloro-terbutoxycarbonyl)-10-acyloxy-taxanes répondant à la formule générale **3c** suivante : 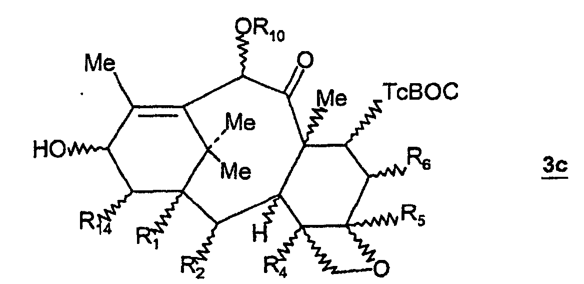 dans laquelle
R₁, R₂, R₄, R₅, R₆, R₇ et R₁₄ sont définis précédemment, et
R₁₀ représente un radical acyle de formule O-CO-R, R étant défini précédemment,
sont ensuite préparés par addition lente à température ambiante du chlorure d'acyle correspondant (1 à 1,2 équivalents) dilué dans un solvant approprié sur une solution fortement agitée du dérivé de 7-(2,2,2-trichloro-terbutoxycarbonyl)-10-hydroxy-taxanes de formule 3b, en présence de pyridine, et/ou d'une pyridine substituée encombrée, comme par exemple la 4-pyrolidinopyridine ou la diméthylaminopyridine.

Pour ces deux réactions, le solvant approprié est un solvant non hydroxylé, en particulier un halogénure d'alkyle, comme par exemple le chlorure de méthylène, le chloroforme ou le dichloroéthane.

Les taxanes **3c** protégés en 7 obtenus précédemment peuvent être employés pour l'hémisynthèse de taxanes, par estérification de l'hydroxyle en 13 avec un précurseur de chaîne latérale de taxane approprié, pour obtenir un dérivé de taxane de formule générale **3d** 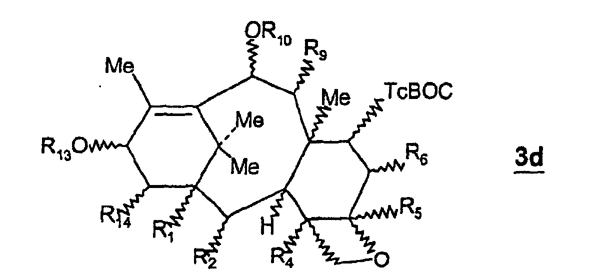 dans laquelle
R₁, R₂, R₄, R₅, R₆, R₇, R₁₀ et R₁₄ sont définis précédemment, et
R₁₃ représente un radical précurseur de chaîne latérale de taxane,
puis par la déprotection sélective de l'hydroxyle en 7, éventuellement accompagnée et/ou précédée de l'ouverture et/ou la transformation et/ou la déprotection du précurseur de chaîne latérale pour obtenir le taxane recherché.

On emploiera en particulier les précurseurs de chaîne latérale de taxanes décrits dans l'état de la technique (EP-0 253 738, EP-0 336 840, EP-0 336 841, EP-0 495 718, WO 92/09589, WO 94/07877, WO 94/07878, WO 94/07879, WO 94/10169, WO 94/12482, EP-0 400 971, EP-0 428 376, WO 94/14787) ou une oxazolidinone décrite dans la demande de brevet FR-95 12 735. Il s'agit en particulier des précurseurs de chaîne suivants : 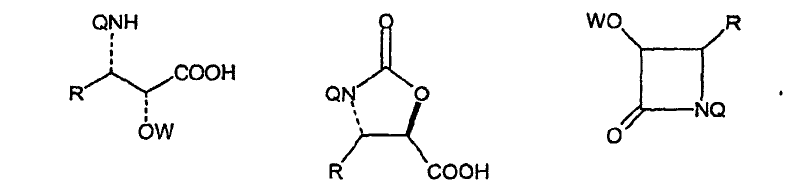 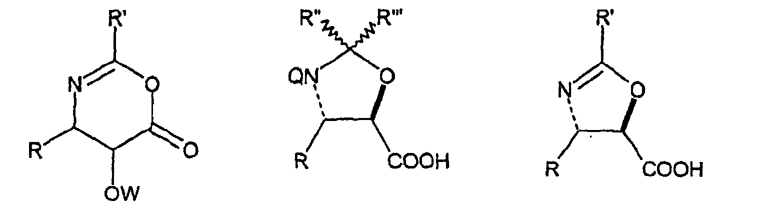 pour lesquels
Q = H, RCO, ROCO,
W = Bz ou un groupe protecteur de la fonction hydroxy GP,
R' = R, OR, SR, X, Si(R)₃,
R" et R"' = R,
R étant défini précédemment,
associés au dérivé de formule **3c** selon les techniques connues dans la littérature (EP-0253738, EP-0336840, EP-0336841, EP-0495718, WO 92/09589, WO 94/07877, WO 94/07878, WO 94/07879, WO 94/10169, WO 94/12482, EP-0400971, EP-0428376, WO 94/14787 ou FR 9512735), pour donner le précurseur direct de taxanes correspondants.

D'une manière avantageuse, on emploiera les précurseurs de chaîne latérale de taxanes suivants : 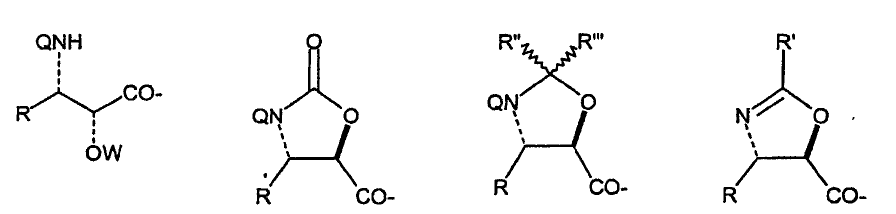 pour lesquels Q, R, R', R", R"' et W sont définis précédemment, en particulier ceux pour lesquels R et R' représentent un aryle, et Q représente un radical aroyle.

S'il s'agit d'une oxazolidinone décrite dans la demande de brevet FR 95-12735, il s'agira de préférence de l'acide N-benzoyl-4-phényl-oxazolidin-2-one carboxylique, en particulier l'isomère (4S,5R), avantageusement obtenue par saponification ménagée de l'ester (+)-menthylique correspondant.

La déprotection de l'hydroxyle en 7 de la baccatine s'effectue par β-élimination, selon les techniques usuelles [P.J. Kocienski, Protecting Groups, Thieme Verlag Ed, p. 7 (1994) et références citées].

Le procédé selon l'invention est décrit d'une manière générale pour des 7,10-dihydroxytaxanes de formule générale **3a** puisqu'il concerne la protection sélective de l'hydroxyle en 7, indépendamment de la nature des substituants R₁, R₂, R₄, R₅, R₆ ou R₁₄. Bien entendu, il est particulièrement adapté à la protection de la 10-désacétylbaccatine de formule générale **2** pour laquelle R₇ et R₁₀ représentent un atome d'hydrogène, intermédiaire-clé dans l'hémisynthèse du PACLITAXEL.

La présente invention concerne donc également un procédé de préparation du PACLITAXEL, obtenu par l'estérification d'un dérivé de la baccatine III de formule générale **2** pour laquelle R₇ représente un groupement protecteur tel que défini ci-dessus, et R₁₀ représente un radical acétyle, avec un précurseur approprié de la chaîne latérale du PACLITAXEL, puis par la déprotection de l'hydroxyle en 7 selon la technique décrite ci-dessus, éventuellement accompagnée et/ou précédée de l'ouverture et/ou la transformation et/ou la déprotection du précurseur de chaîne latérale du PACLITAXEL, pour donner ladite chaîne latérale du PACLITAXEL.

Par alkyle linéaire ou ramifié on entend de préférence selon l'invention un alkyle en C₁-C₆, en particulier choisi parmi les radicaux méthyle, éthyle, propyle, isopropyle, butyle et ses différents isomères ramifiés comme par exemple le ter-butyle, pentyle et hexyle et leurs différents isomères ramifiés. Cette définition s'applique également aux restes alkyles des radicaux alkoxy, aralkyle ou aralkoxy.

Par cycloalkyle, on entend de préférence selon l'invention un cycloalkyle en C₃-C₆, en particulier choisi parmi les radicaux cyclopropyle, cyclobutyle, cyclopentyle ou cyclohexyle.

Par aryle on entend de préférence selon l'invention un radical aromatique ou hétéroaromatique, en particulier choisi parmi les radicaux phényle, naphtyle, anthryle, phénantryle, pyridyle, pyrimidyle, etc.

Enfin, par halogène on entend de préférence le chlore, le brome ou l'iode. Pour les radicaux perhalogénoalkyles, il s'agira de préférence de radicaux perchlorés, en particulier les radicaux trichlorométhyle ou pentachloroéthyle.

D'autres caractéristiques des procédés et intermédiaires selon l'invention apparaîtront à la lumière des exemples ci-après.

### 4ETUDES COMPARATIVES A L'AIDE D'HALOALCOXYCARBONYLES

L'objet principal de cette invention étant de démontrer l'avantage déterminant, en matière de sélectivité, de groupements protecteurs alcoxycarbonyles β-substitués, plus encombrés que le 2,2,2-trichloro-éthoxycarbonyle (Troc), déjà utilisé de façon non sélective dans la protection non sélective des taxanes présentant au moins deux hydroxyles libres en 7 et 10 (EP-A-0336841), l'étude cinétique comparative a été réalisée par rapport à ce dernier.

A titre de comparaison, et sans limiter la portée de la présente invention, nous avons sélectionné comme exemple le 2,2,2-trichloro-ter-butoxycarbonyle (TCBoc), groupement protecteur sensiblement plus encombré que le Troc, en utilisant comme exemple de substrat la 10-désacétylbaccatine III, précurseur habituel de l'hémisynthèse du PACLITAXEL et du DOCETAXEL.

Les conditions réactionnelles utilisées dans cet exemple d'étude cinétique sont les suivantes :
- Température : 38°C
- Solvant : dichlorométhane
- Chloroformiate : 1,1 à 1,5 équivalents molaires
- Durée totale de l'étude : 16 heures
- Catalyseurs : pyridine, 5 équivalents molaires, 4-pyrolidinopyridine, 1,4 équivalents molaires
- Modalités : agitation énergique, addition lente de chloroformiate (1 équivalent molaire par heure).

Le suivi analytique quantitatif a été effectué par chromatographie liquide à haute performance.

Les résultats de cette étude cinétique comparative sont repris sur le Tableau I ci-après.

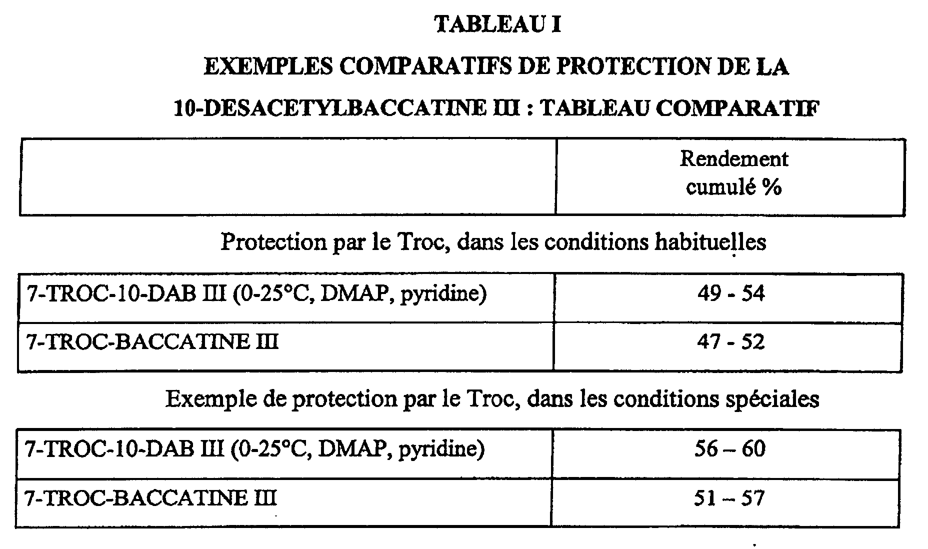

Exemple de protection par le TCboc, dans les conditions spéciales

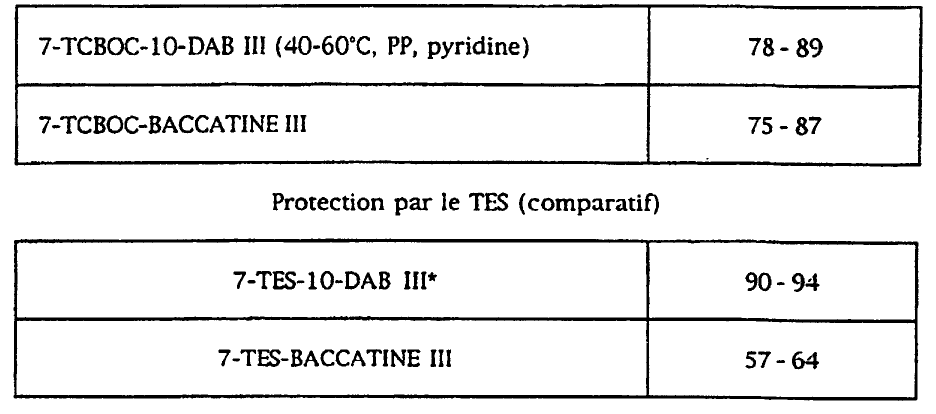

Abréviations : TROC = 2,2,2-Trichloroéthoxycarbonyl ; TCBOC = 2,2,2-Trichloroterbutoxy-carbonyl ; TES = Triéthylsilyl ; DMAP = Diméthylaminopyridine ; PP = 4 -pyro-lidinopyridine ; DAB = désacétylbaccatine.
* 84-86 % selon la littérature [J.-N. Denis, A. Greene, *J. Am. Chem. Soc.,* 5917 (1988)].

Les résultats de cette étude comparative montrent sans équivoque que les nouveaux groupements protecteurs selon l'invention employés pour la synthèse de la baccatine III protégée en 7, permettent d'obtenir de la 10-désacétylbaccatine III protégée en 7 avec une sélectivité voisine de celle du triéthylsilyle, seul groupement employé dans l'état de la technique.

Lorsque l'on effectue ensuite l'acylation sélective de l'hydroxyle en position 10, on obtient alors des rendements supérieurs à ceux obtenus avec une 7-triéthylsilyle-10-désacétylbaccatine III.

On obtient donc un rendement global de synthèse de la baccatine III protégée en 7 à partir de la 10-désacétylbaccatine supérieur à celui obtenu pour le procédé de l'état de la technique (75-87 % contre 57-64 %).

### PARTIE EXPERIMENTALE

### EXEMPLE 1

### 7-O-(2,2,2-Trichloro-t-butoxycarbonyl )-10-désacétylbaccatine III

A une solution agitée à 40°C sous atmosphère inerte de 500 mg (0,919 mmol) de 10-désacétylbaccatine III et 0,19 g (1,29 mmol) de 4-pyrolidinopyridine dans 5 ml de dichlorométhane sec, on additionne une solution de 0,26 g (1,10 mmol) de chlorure de 2,2,2-trichloro-t-butoxycarbonyle dans 2 ml de dichlorométhane en 50 min. Après 1 h de réaction supplémentaire, et retour à température ambiante, la solution organique est lavée avec une solution aqueuse d'HCl 2 % (5ml), lavée avec de l'eau osmosée (2 x 5 ml), séchée sur MgSO₄ et concentrée sous pression réduite. Après chromatographie du produit brut sur gel de silice (15-40 µm) (éluant : cyclohexane-acétate d'éthyle, 60/40) on obtient la 7-O-(2,2,2-trichloro-t-butoxycarbonyl)-10-désacétylbaccatine III sous la forme d'une poudre blanche (Rdt = 89 %).

Le produit obtenu présente les caractéristiques suivantes :
- RMN ¹H 400 MHz (CD CI₃) (δ ppm) : 8,10 (2H, d, J = 7,3 Hz) ; 7,62 (1H, t, J = 7,3 Hz) ; 7,49 (2H, t, J = 7,6 Hz) ; 5,64 (1H, d, J = 6,8 Hz) ; 5,50 (1H, d) ; 5,39 (1H, dd, J = 10,6 et 7,3 Hz) ; 4,97 (1H, d, J = 8,6 Hz) ; 4,89 (1H, m) ; 4,34 et 4,20 (2H, 2d, J = 8,4 Hz) ; 4,09 (1H, d) ; 4,06 (1H, d) ; 2,60 (1H, m) ; 2,31 (3H, s) ; 2,29 (1H, m) ; 2,13 (3H, s) et (1H, m) ; 2,06 (1H, m) ; 1,90 (6H, s) ; 1,85 (3H, s) ; 1,09 (3H, s); 1,06 (3H, s).

### EXEMPLE 2

### 7-O-(2,2,2-Trichloro-t-butoxycarbonyl) baccatine III

A une solution agitée à température ambiante sous atmosphère inerte de 260 mg (0,347 mmol) de 7-O(2,2,2-trichloro-t-butoxycarbonyl)-10-désacétylbaccatine III et 127,5 mg (1,04 mmol) de 4-diméthylaminopyridine dans 2,5 ml de dichlorométhane sec, on ajoute 50 µl (0,695 mmol) de chlorure d'acétyle. Après 1 h de réaction à température ambiante, la phase organique est lavée avec une solution aqueuse d'HCl 2 % jusqu'à obtention d'un pH = 6, séchée sur MgSO₄ et concentrée sous pression réduite. Après chromatographie du résidu obtenu sur gel de silice (15-40 µm) (éluant : cyclohexane-acétate d'éthyle, 6/4), on obtient le 7-O-(2,2,2-trichloro-t-butoxycarbonyl) baccatine III à l'état solide (Rdt = 96 %).

Le composé obtenu présente les caractéristiques suivantes :
- RMN ¹H 400 MHz (CDC13) (δ ppm) : 8,10 (2H, d, J = 7,6 Hz) ; 7,61 (1H, t, J = 7,4 Hz) ; 7,48 (2H, t, J = 7,7 Hz) ; 6.52 (1H, s) ; 5,65 (1H, d, J = 6,9 Hz) ; 5,39 (1H, dd, J = 10,4 et 7,3 Hz) ; 4,96 (1H, d, J = 8,9 Hz) ; 4,86 (1H, m) ; 4,32 et 4,17 (2H, 2d, J = 8,4 Hz) ; 4,01 (1H, d, J = 6,9 Hz) ; 2,69 (1H, m) ; 2,30 (2H, m) ; 2,29 (3H, s) ; 2,16 (3H, s) ; 2,14 (3H, s) ; 2,07 (1H, d, J = 4,8) ; 1,97 (1H, m) ; 1,95 et 1,91 (6H, 2s) ; 1,80 (3H, s) ; 1,61 (1H, s) ; 1,15 (3H, s) ; 1,07 (3H, s).

### EXEMPLE 3 (comparatif)

### 7-O-trichloroéthoxycarbonyl-10-désacétylbaccatine III

A une solution agitée à 40°C sous atmosphère inerte de 500 mg (0,919 mmol) de 10-désacétylbaccatine III et 0,19 g (1,29 mmol) de 4-pyrolidinopyridine dans 5 ml de dichlorométhane sec, on additionne une solution de 0,152 ml (1,10 mmol) de chlorure de trichloroéthoxycarbonyle dans 2 ml de dichlorométhane en 40 min. Après 1 h de réaction supplémentaire, et retour à température ambiante, la solution organique est lavée avec une solution aqueuse d'HCl 2 % (5ml), lavée avec de l'eau osmosée (2 x 5 ml), séchée sur MgSO₄ et concentrée sous pression réduite (Rdt HPLC = 57 %). Après chromatographie du produit brut sur gel de silice (15-40 µm) (éluant : cyclohexane-acétate d'éthyle, 60/40) on obtient la 7-O-trichloroéthoxycarbonyl-10-désacétylbaccatine III sous la forme d'une poudre blanche.

Le produit obtenu présente les caractéristiques suivantes :
- RMN ¹H 400 MHz (CDCI₃) (δ ppm) : 8,10 (2H, d, J = 7 Hz) ; 7,62 (1H, t, J = 7,4 Hz) ; 7,49 (2H, t, J = 7,6 Hz) ; 5,65 (1H, d, J = 6,9 Hz) ; 5,44 (1H, dd, J = 10,8 et 7,3 Hz) ; 5,39 (1H, d) ; 4,98 (1H, d, J = 7,5 Hz) ; 4,89 (1H, m) ; 4,84 et 4,70 (2H, 2d, J = 11.9 Hz) ; 4,35 et 4,20 (2H, 2d, J = 8,4 Hz) ; 4,10 (1H, d, J = 7Hz) ; 4,01 (1H, d, J = 1.8 Hz) ; 2,64 (1H, m) ; 2,31 (3H, s) ; 2,29 (1H, m) ; 2,11 (3H, d) ; 2.05 (2H, m) ; 1,89 (3H, s) ; 1,09 (3H, s) ; 1,07 (3H, s).

### EXEMPLE 4

### 7-O-trichloroéthoxycarbonylbaccatine III (comparatif)

A une solution agitée à température ambiante sous atmosphère inerte de 1,70 g (2,36 mmol) de 7-O-trichloroéthoxycarbonyl-10-désacétylbaccatine III et 0,96 ml (12 mmol) de pyridine dans 17 ml de dichlorométhane sec, on a joute 0,68 ml (0,695 mmol) de chlorure d'acétyle. Après 3 h de réaction à température ambiante, la phase organique est lavée avec de l'eau (2 x 20 ml), séchée sur MgSO₄ et concentrée sous pression réduite. Après chromatographie du résidu obtenu sur gel de silice (15-40 µm) (éluant : cyclohexane-acétate d'éthyle, 6/4) on obtient 1,38 g de 7-O-trichloroéthoxycarbonylbaccatine III à l'état solide (Rdt = 75 %).

Le composé obtenu présente les caractéristiques suivantes :
- RMN ¹H 400 MHz (CDCl₃) (δ ppm) : 8,11 (2H, d, J = 7,1 Hz) ; 7,62 (1H, t, J = 7,4 Hz) ; 7,49 (2H, t, J = 7,6 Hz) ; 6,39 (1H, s) ; 5,64 (1H, d, J = 6,9 Hz) ; 5,61 (1H, dd, J = 10,7 et 7,2 Hz) ; 5,04 et 4,65 (2H, 2d, J = 12 Hz) ; 4,99 (1H, d, J = 8,2 Hz) ; 4,87 (1H, m) ; 4,33 et 4,16 (2H, 2d, J = 8,4 Hz) ; 4,02 (1H, d, J = 6,9 Hz) ; 2,64 (1H, ddd, J = 14,4, 9,5 et 7,2 Hz) ; 2,30 (3H, s) et (2H, m) ; 2,17 (3H, s) ; 2,13 (3H, d, J = 0,8 Hz) ; 2,04 (1H, m) ; 1,83 (3H, s) ; 1,63 (1H, s) ; 1,14 (3H, s) ; 1,09 (3H, s).

### EXEMPLE 5

### 13-O-[[(4S,5R)-2,4-Diphenyl-4,5-dihydrooxazol-5-yl)carbonyIl-7-O-(2,2,2-trichloro-t-butoxycarbonyl) baccatine III

A une solution agitée à température ambiante et sous atmosphère inerte de 3,38 g (12,7 mmol) d'acide (4S,5R)-2,4-diphényl-4,5-dihydrooxazol-5-carboxylique dans 60 ml de toluène antydre, on ajoute 2,61 g (12,7 mmol) de dicyclohexylcarbodiimide. Après 5 min d'agitation, on ajoute 5 g (6,33 mmol) de 7-O-(2,2,2-trichloro-t-butoxycarbonyl) baccatine III et 0,77 g (6,33 mmol) de 4-diméthylaminopyridine, et le mélange est agité à température ambiante pendant 15 min. Après élimination des insolubles par filtration, la phase organique est concentrée sous pression réduite, et le résidu obtenu est purifié par chromatographie sur gel de silice (15-40 µm) (éluant : cyclohexane-acétate d'éthyle, 9/1).

On obtient ainsi 6,2 g du composé cité en titre sous la forme d'un solide blanc (Rdt = 94 %), et qui présente les caractéristiques suivantes :
- RMN ¹H 400 MHz (CDCl₃) (δ ppm) : 8,18 (2H, d, J = 7,2 Hz) ; 8,07 (2H, d, J = 7,6 Hz) ; 7,64 (1H, t, J = 7,4 Hz) ; 7,60 (1H, t, J = 7,3 Hz) ; 7,52 (4H, m) ; 7,39 (5H, m); 6,47 (1H, s); 6,24 (1H, t, J = 8,4 Hz) ; 5,70 (1H, d, J = 7Hz); 5,59 (1H, d, J = 7,3 Hz) ; 5,35 (1H, dd, J = 10,4 et 7,2) ; 4,93 (2H, d, J = 7,3 Hz) ; 4,29 et 4,17 (2H,2d, J = 8,5 Hz) ; 3,96 (1H, d, J = 6,9 Hz) ; 2,71 (1H, m) ; 2,37 (1H, dd, J = 15,1 et 9,2 Hz) ; 2,28 (1H, dd, J = 15,1 et 8,8 Hz) ; 2,13 (3H, s) ; 2,01 (6H, s) ; 1,95 et 1,93 (6H, 2s) ; 1,80 (3H, s) ; 1,72 (1H, s) ; 1,23 (3h, s) ; 1,18 (3H, s).

### EXEMPLE 6

### 13-O-[(2R,3S)-O-Benzoyl-3-phénylisosérin-1-yl]-7-O-(2,2,2-trichloro-t-butoxycarbonyl) baccatine III

A une solution agitée à température ambiante et sous atmosphère inerte de 100 mg (0,0963 mmol) de 13-O-[[(4S,5R)-2,4-diphényl-4,5-dihydrooxazol-5-yl]-carbonyl]-7-O-(2,2,2-trichloro-t-butoxycarbonyl) baccatine III dans un mélange de tétrahydrofurane (1 ml) et de méthanol (1 ml), on additionne 0,2 ml (0,2 mmol) d'une solution aqueuse d'HCl 1M, et le mélange réactionnel est agité à température ambiante pendant 14 h 30. Après addition de 15 mg (0,2 mmol) d'hydrogénocarbonate de sodium solide suivie d'une agitation de 10 min, le milieu réactionnel est extrait à l'acétate d'éthyle (2 x 2 ml), et la phase organique est lavée à l'eau, séchée sur MgSO₄, concentrée sous pression réduite.

On isole ainsi 102 mg du composé cité en titre (Rdt brut = quantitatif), que l'on utilisera tel quel dans l'étape suivante, et qui présente les caractéristiques suivantes :
- RMN ¹H 400 MHz (DMSO-d₆) (δ ppm) : 8,15 (2H, d, J = 7,9 Hz) ; 7,97 (2H, d, J = 7,7 Hz) ; 7,75 (2H, t, J = 7,4 Hz) ; 7,64 (1H, t, J = 7,7 Hz) ; 7,59 (1H, t, J = 7,7 Hz) ; 7,48 (2H, d, J = 7,3 Hz) ; 7,42 (2H, t, J = 7,5 Hz) ; 7,42 (2H, t, J = 7,5 Hz) ; 7,19 (1H, t, J = 7Hz) ; 6,34 (1H, s) ; 5,89 (1H, t, J = 8,9 Hz ) ; 5,47 (1H, d, J = 7 Hz) ; 5,28 (1H, dd, J = 10,5 e t7,3) ; 5,16 (1H, d, J = 6,8 Hz) ; 4,98 (1H, d, J = 9,5 Hz) ; 4,80 (1H, s) ; 4,41 (1H, d, J = 6,8) ; 4,06 (2H, s large) ; 3,72 (1H, d, J = 7 Hz) ; 2,26 (3H, s) ; 2,07 (3H, s) ; 2,0 à 1,6 (4H, m) ; 1,86 (6H, s) ; 1,83 (3H, s) ; 1,64 (3H, s) ; 1,05 (3H, s) ; 1,01 (3H, s).

### EXEMPLE 7

### 7-O-(2,2,2-trichloro-t-butoxycarbonyl)-taxol

A une solution agitée à température ambiante et sous atmosphère inerte de 90 mg (0,0852 mmol) de 13-O-[(2R,3S)-O-Benzoyl-3-phénylisosérin-1-yl]-7-O-(2,2,2-trichloro-t-butoxycarbonyl) baccatine III dans un mélange de tétrahydrofurane (4 ml) et de méthanol (4 ml), on additionne 0,25 ml d'une solution aqueuse saturée d'hydrogénocarbonate de sodium, et le mélange réactionnel est agité à température ambiante pendant 48 h. Après extraction du milieu réactionnel à l'acétate d'éthyle (2 x 5 ml), la phase organique séparée est lavée à l'eau (5 ml), séchée sur MgSO₄, concentrée sous pression réduite, et le résidu obtenu est purifié par chromatographie sur gel de silice (15-40 µm) (éluant : cyclohexane-acétate d'éthyle, 6/4).

On obtient ainsi 63 mg du composé cité en titre sous la forme d'un solide blanc (Rdt = 70 %), et qui présente les caractéristiques suivantes :
- RMN ¹H 400 MHz (CDCl₃) (δ ppm) : 8,12 (2H, d, J = 7,4 Hz) ; 7,75 (2H, d, J = 7,3 Hz) ; 7,61 (1H, t, J = 7,4 Hz) ; 7,50 (5H, m) ; 7,38 (5H, m) ; 7,19 (1H, d, J = 9 Hz) ; 6,47 (1H, s) ; 6,18 (1H, t, J = 8,6 Hz) ; 5,79 (1H, dd, J = 8,9 et 2,2 Hz) ; 5,70 (1H, d, J = 6,8 Hz) ; 5,33 (1H, dd, J = 10,3 et 7,2) ; 4,93 (1H, d, J = 9 Hz) ; 4,80 (1H, d, J = 2,4 Hz) ; 4,31 et 4,21 (2H, 2d, J = 8,5 Hz) ; 3,91 (1H, d, J = 6,8 Hz) ; 2,66 (1H, m) ; 2,38 (3H, s) ; 2,33 (2H, m) ; 2,13 (3H, s) ; 1,99 (1H, m) ; 1,94 (3H, s) ; 1,90 (6H, s) ; 1,81 (3H, s) ; 1,19 (3H, s) ; 1,18 (3H, s).

### EXEMPLE 8

### 13-O-[[(4S,5R)-2,4-Diphényl-4,5-dihydrooxazol-5yl]carbonyl]-baccatine III

A une solution agitée à température ambiante et sous atmosphère inerte de 390 mg (0,376 mmol) de 13-O-[[(4S,5R)-2,4-Diphényl-4,5-dihydrooxazol-5-yl] carbonyl]-7-O-(2,2,2-trichloro-t-butoxycarbonylbaccatine III dans 10 ml d'acétate d'éthyle, on ajoute 300 µl (5,26 mmol) d'acide acétique et 221 mg (3,38 mmol) de poudre de zinc. Après 1 h 15 d'agitation à 30°C et contrôle par C. C. M., la phase organique après filtration est lavée avec de l'eau osmosée (5 ml), avec une solution aqueuse saturée d'hydrogénocarbonate de sodium (2 x 5 ml), à nouveau avec de l'eau (2 x 5 ml), séchée sur MgSO₄ et concentrée sous pression réduite.

On isole ainsi 314 mg du composé cité en titre (Rdt brut = quantitatif), que l'on utilisera tel quel dans l'étape suivante, et qui présente les caractéristiques suivantes :
- RMN ¹H 400 MHz (CDCl₃) (δ ppm) : 8,18 (2H, d, J = 7,3 Hz) ; 8,07 (2H, d, J = 7,4 Hz) ; 7,64 (1H, t, J = 7,4 Hz) ; 7,60 (1H, t, J = 7,3 Hz) ; 7,52 (2H, t, J = 8,1 Hz) ; 7,50 (2H, t, J = 7,8 Hz) ; 7,39 (5H, m) ; 6,27 (1H, s) ; 6,24 (1H, t, J = 8,5 Hz) ; 5,67 (1H, d, J = 7.1Hz) ; 5,59 (1H, d, J = 6,9 Hz) ; 4,96 (1H, d) ; 4,95 (1H, d, J = 6,9 Hz) ; 4,29 et 4,15 (2H, 2d, J = 8,4 Hz) ; 3,82 (1H, d, J = 7 Hz) ; 2,57 (1H, ddd, J = 15,9,6 et 6,8 Hz) ; 2,48 (1H, d, J = 4 Hz) ; 2,38 (1H, dd, J = 15,3 et 9 Hz) ; 2,27 (1H, dd, J = 15,3 et 8,7 Hz) ; 2,24 (3H, s) ; 2,04 (3H, s) ; 1,89 (3H, s) ; 1,88 (1H, m) ; 1,75 (1H, s) ; 1,67 (3H, s) ; 1,26 (3H, s) ; 1,15 (3H, s).

### EXEMPLE 9

### Préparation du paclitaxel

### a) à partir de 13-O-[[(4S,SR)-2,4-diphényl-4,5-dihydrooxazol-5-yl]-carbonyl]-7-O-(2,2,2-trichloro-t-buloxycarbonyl) baccatine III

A une solution agitée à température ambiante et sous atmosphère inerte de 15 mg (0,0148 mmol) de 13-O-[[(4S,SR)-2,4-diphényl-4,5-dihydrooxazol-5-yl]-carbonyl]-7-O-(2,2,2-trichloro-t-butoxycarbonyl) baccatine III dans un mélange de tétrahydrofurane (0,18 ml) et de méthanol (0,18 ml), on additionne 90 µl (0,09 mmol) d'une solution aqueuse d'HCl 1M, et le mélange réactionnel est agité à température ambiante pendant 8 h. Après addition de 0,6 ml d'une solution aqueuse saturée d'hydrogénocarbonate de sodium, la solution est maintenue homogène par addition de 1 ml de tétrahydrofurane et 1 ml d'eau, et le milieu réactionnel est agité 1 h 30 supplémentaire. Après addition de 2,5 ml d'acétate d'éthyle et 2,5 ml d'eau osmosée, la phase aqueuse résiduelle est extraite à l'acétate d'éthyle (2,5 ml). Les phases organiques réunies sont séchées sur MgSO₄, et concentrées sous pression réduite.

On obtient ainsi 14 mg de 7-O-(2,2,2-trichloro-t-butoxycarbonyl)-taxol à l'état brut (Rdt = 93 %) que l'on utilise sans autre purification dans l'étape suivante.

A une solution agitée à température ambiante de 13 mg (0,0128 mmol) de 7-O-(2,2,2-trichloro-t-butoxycarbonyl)-taxol dans 2 ml d'acétate d'éthyle, on ajoute 30 µl (0,525 mmol) d'acide acétique et 22,5 mg (0,344 mmol) de poudre de zinc. Après 2 h 30 d'agitation à température ambiante et contrôle par C . C . M., et après dilution du milieu réactionnel avec 3 ml d'acétate d'éthyle, la phase organique est lavée avec de l'eau osmosée (1 ml), avec une solution aqueuse saturée d'hydrogénocarbonate de sodium (1 ml), à nouveau avec de l'eau, séchée sur MgSO₄ et concentrée sous pression réduite.

Après chromatographie du produit brut sur gel de silice (15-40 µm) (éluant : cyclohexane-acétate d'éthyle, 6/4) on isole ainsi 9,5 mg de PACLITAXEL à l'état cristallisé (Rdt = 89 %).

### b) à partir de 13-O-[[(4S,5R)-2,4-diphényl-4,5-dihydrooxazol-5-yl)-carbonyl)]-baccatine III

A une solution agitée à température ambiante et sous atmosphère inerte de 290 mg (0,347 mmol) de 13-O-[[(4S,5R)-2,4-diphényl-4,5-dihydrooxazol-5-yl]-carbonyl]-baccatine III dans un mélange de tétrahydrofurane (4 ml) et de méthanol (4 ml), on additionne 0,7 ml (0,7 mmol) d'une solution aqueuse d'HCl 1M, et le mélange réactionnel est agité à température ambiante pendant 4 h. Après addition de 6 ml d'une solution aqueuse saturée d'hydrogénocarbonate de sodium, la solution est maintenue homogène par addition de 10 ml d'eau, et le milieu réactionnel est agité 3 h 30 supplémentaires. Après addition de 20 ml d'acétate d'éthyle et 10 ml d'eau osmosée, la phase aqueuse résiduelle est extraite à l'acétate d'éthyle (20 ml). Les phases organiques réunies sont séchées sur MgSO₄, et concentrées sous pression réduite.

Après chromatographie du produit brut sur gel de silice (15-40 µm) (éluant : cyclohexane-acétate d'éthyle, 5/5) on isole ainsi 270 mg de PACLITAXEL à l'état cristallisé (Rdt = 93 %).

## Revendications

1. Procédé de préparation de dérivés 7-(2,2,2-trichloro-terbutoxycarbonyl)-10-hydroxy-taxanes répondant à la formule **3b** suivante : 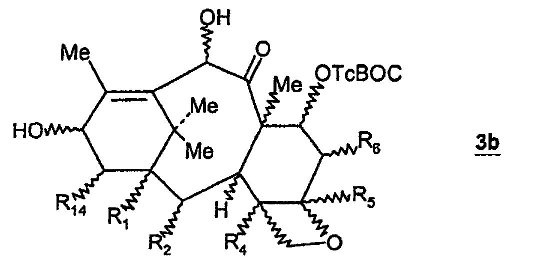 dans laquelle
R₁, R₂, R₄, R₅, R₆ et R₁₄ représentent indépendamment l'un de l'autre un radical Q, avec :
Q = H, OH, R, OR, SH, SR, OCOR, OCOOR, HCO, X, et
X = halogène,
R représente, indépendamment l'un de l'autre un radical alkyle ou alkylène linéaire ou ramifié, un radical perhalogénoalkyle, un radical hétéroalkyle ou hétéroalkylène linéaire ou ramifié, un radical cycloalkyle ou cycloalkylène, un radical hétérocycloalkyle ou hétérocycloalkylène, un radical aryle, aralkyle, les radicaux pouvant être substitués, en particulier par un ou plusieurs halogènes,
par addition lente à une température supérieure à la température ambiante, de préférence comprise entre 20 et 80°C, du chlorure de 2,2,2-trichloro-terbutoxycarbonyl dilué dans un solvant approprié, sur une solution fortement agitée du 7-10-dihydroxytaxane de formule générale **3a** 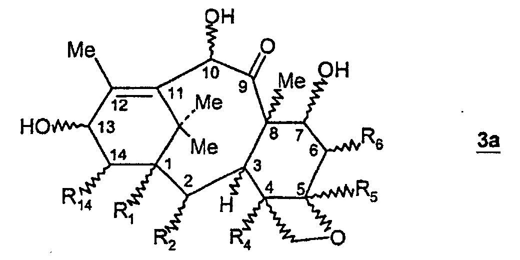 dans laquelle
R₁, R₂, R₄, R₅, R₆ et R₁₄ sont définis ci-dessus,
en présence de pyridine et/ou d'une pyridine substituée encombrée, comme par exemple la 4-pyrolidinopyridine ou la diméthyl-aminopyridine.

2. Procédé selon la revendication 1, **caractérisé en ce que** le 10-hydroxy-taxane de formule **3a** est de la 10-désacétylbaccatine de formule générale **2**

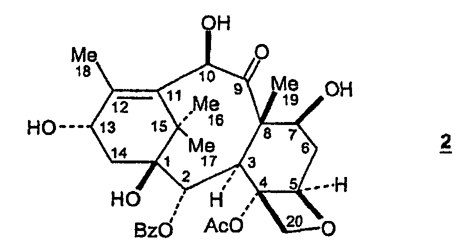

3. Procédé selon l'une des revendications 1 à 2, **caractérisé en ce que** l'estérification est conduite avec 1 à 1,5 équivalents de chlorure de 2,2,2-trichloro-terbutoxycarbonyl par rapport au 7-10-dihydroxytaxane de formule générale **3a**.

4. Procédé selon l'une des revendications 1 à 3, **caractérisé en ce que** l'on prépare ensuite un dérivé 7-(2,2,2-trichloro-terbutoxycarbonyl)-10-acyloxy-taxane de formule générale **3c** suivante : 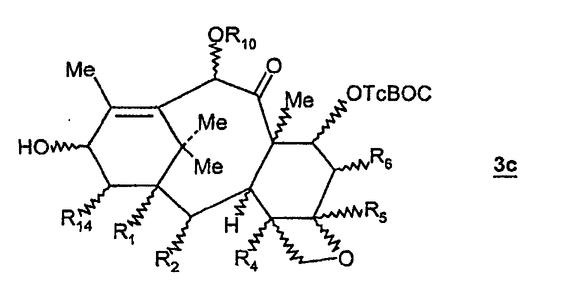 dans laquelle
R₁, R₂, R₄, R₅, R₆ et R₁₄ sont définis à la revendication 1, et
R₁₀ représente un radical acyle de formule O-CO-R, R étant défini à la revendication 1,
par addition lente à température ambiante du chlorure d'acyle correspondant dilué dans un solvant approprié sur une solution fortement agitée du dérivé de 7-(2,2,2-trichloro-terbutoxycarbonyl)-10-hydroxy-taxane de formule **3b** défini dans la revendication 1, en présence de pyridine, et/ou d'une pyridine substituée encombrée, comme par exemple la 4-pyrolidinopyridine ou la diméthylaminopyridine.

5. Procédé selon la revendication 4, **caractérisé en ce que** R représente un groupe alkyle, en particulier méthyle.

6. Procédé selon l'une des revendications 1 à 4, **caractérisé en ce que** le solvant approprié est un solvant non hydroxylé, en particulier un halogénure d'alkyle, comme par exemple le chlorure de méthylène, le chloroforme ou le dichloroéthane.

7. Procédé selon l'une des revendications 1 à 4, **caractérisé en ce que** les taxanes de formule générale **3c** définis dans la revendication 4, sont ensuite employés pour l'hémisynthèse de taxanes, par estérification de l'hydroxyle en 13 avec un précurseur de chaîne latérale de taxane approprié, pour obtenir un dérivé de taxane de formule générale **3d** 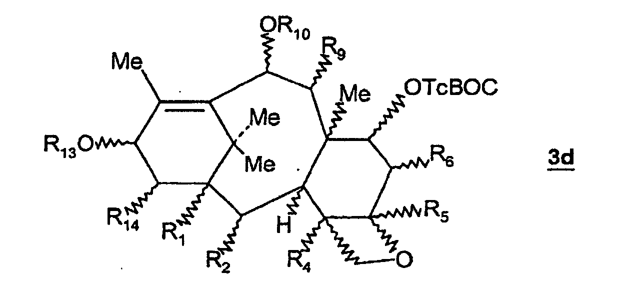 dans laquelle
R₁, R₂, R₄, R₅, R₆, R₁₀ et R₁₄ sont définis à la revendication 1, et
R₁₃ représente un radical précurseur de chaîne latérale de taxane, puis par la déprotection sélective de l'hydroxyle en 7, éventuellement accompagnée et/ou précédée de l'ouverture et/ou la transformation et/ou la déprotection du précurseur de chaîne latérale pour obtenir le taxane recherché.

8. Procédé selon la revendication 7, **caractérisé en ce que** R₁₃ est le précurseur de chaîne latérale du PACLITAXEL.

9. Procédé selon l'une des revendications 7 ou 8, **caractérisé en ce que** la déprotection de l'hydroxyle en 7 s'effectue par β-élimination.

10. Dérivés 7-(2,2,2-trichloro-terbutoxycarbonyl)-10-hydroxy-taxanes répondant à la formule **3b** suivante : 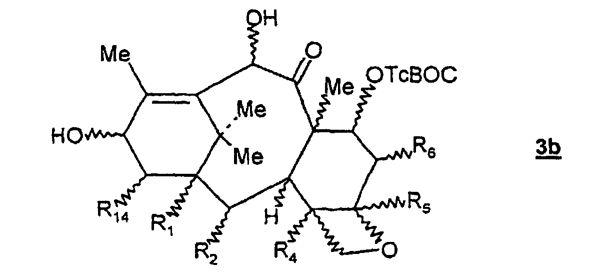 dans laquelle
R₁, R₂, R₄, R₅, R₆ et R₁₄ sont définis dans la revendication 1.

11. Dérivés de formule générale **3c** suivante : 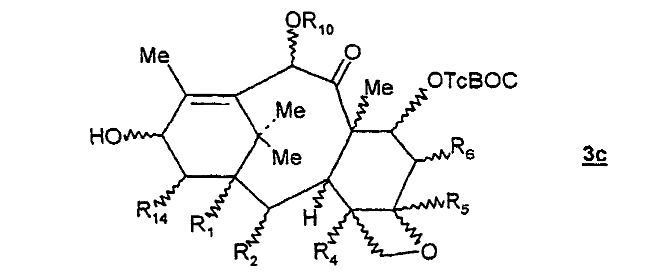 dans laquelle
R₁, R₂, R₄, R₅, R₆ et R₁₄ sont définis dans l'une des revendications précédentes, et R₁₀ est défini dans la revendication 4.

## Patentansprüche

1. Verfahren zur Herstellung von 7-(2,2,2-Trichlor-tert.-butoxycarbonyl)-10-hydroxy-taxan-Derivaten der folgenden Formel 3b: 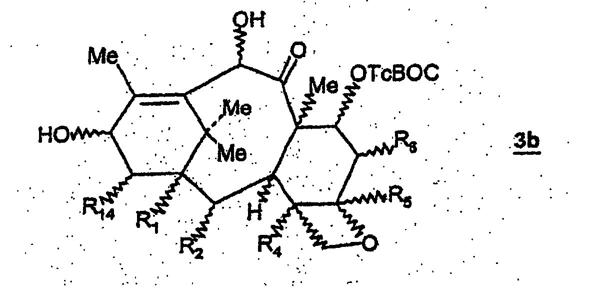 in der
R₁, R₂, R₄, R₅, R₆ und R₁₄, unabhängig voneinander, einen Rest Q darstellen, mit
Q = H, OH, R, OR, SH, SR, OCOR, OCOOR, HCO, X, und
X = Halogen,
R, unabhängig voneinander, einen linearen oder verzweigten Rest Alkyl oder Alkylen, einen Rest Perhalogenalkyl, einen linearen oder verzweigten Rest Heteroalkyl oder Heteroalkylen, einen Rest Cycloalkyl oder Cycloalkylen, einen Rest Heterocycloalkyl oder Heterocycloalkylen, einen Rest Aryl, Aralkyl bedeutet, wobei die Reste substituiert sein können, insbesondere durch ein oder mehrere Halogene,
durch langsame Zugabe bei einer Temperatur von höher als der Umgebungstemperatur, vorzugsweise zwischen 20 °C und 80 °C, von in einem geeigneten Lösungsmittel verdünntem 2,2,2-Trichlor-tert.butoxycarbonyl-chlorid zu einer kräftig gerührten Lösung von 7,10-Dihydroxytaxan der allgemeinen Formel 3a 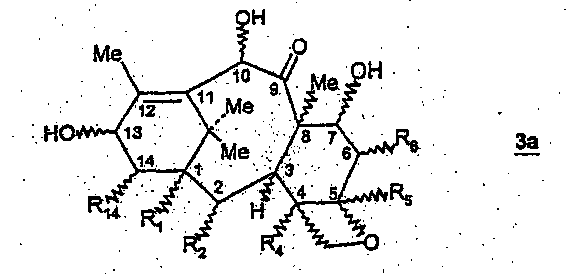 in der R₁, R₂, R₄, R₅, R₆ und R₁₄ wie oben definiert sind, in Anwesenheit von Pyridin und/oder einem substituierten, sterisch sperrigen Pyridin, wie beispielsweise 4-Pyrrolidino-pyridin oder Dimethylaminopyridin.

2. Verfahren nach Anspruch 1, **dadurch gekennzeichnet, daß** das 10-Hydroxy-taxan der allgemeinen Formel 3a das 10-Desacetylbaccatin der allgemeinen Formel 2 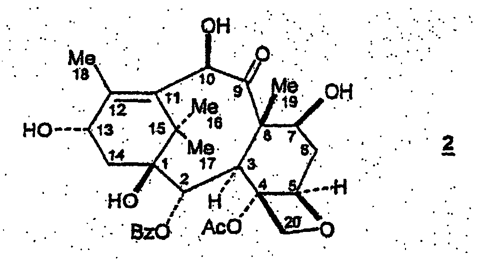 ist.

3. Verfahren nach einem der Ansprüche 1 bis 2, **dadurch gekennzeichnet, daß** die Veresterung mit 1 bis 1,5 Aquivalenten 2,2,2-Trichlor-tert.-butoxycarbonyl-chlorid durchgeführt wird, bezogen auf das 7,10-Dihydroxytaxan der allgemeinen Formel 3a.

4. Verfahren nach einem der Ansprüche 1 bis 3, **dadurch gekennzeichnet, daß** man anschließend ein 7-(2,2,2-Trichlor-tert.-butoxycarbonyl)-10-acyloxy-taxan-Derivat der folgenden der allgemeinen Formel 3c 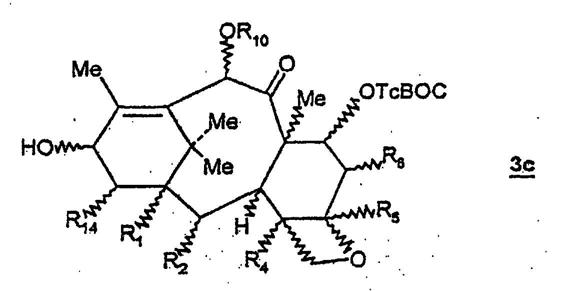 in der
R₁, R₂, R₄, R₅, R₆ und R₁₄ wie in Anspruch 1 definiert sind, und
R₁₀ einen Rest Acyl der Formel O-CO-R darstellt, worin R wie in Anspruch 1 definiert,
herstellt, durch langsame Zugabe bei Umgebungstemperatur von dem entsprechenden, in einem geeigneten Lösungsmittel verdünntem Acylchlorid zu einer kräftig gerührten Lösung des 7-(2,2,2-Trichlor-tert.-butoxycarbonyl)-10-hydroxy-taxan-Derivates der Formel 3b, wie in Anspruch 1 definiert, in Anwesenheit von Pyridin und/oder einem substituierten, sterisch sperrigen Pyridin, wie beispielsweise 4-Pyrrolidino-pyridin oder Dimethylaminopyridin.

5. Verfahren nach Anspruch 4, **dadurch gekennzeichnet, daß** R eine Gruppe Alkyl darstellt, insbesondere Methyl.

6. Verfahren nach einem der Ansprüche 1 bis 4, **dadurch gekennzeichnet, daß** das geeignete Lösungsmittel ein nicht hydroxyliertes Lösungsmittel ist, insbesondere ein Alkylhalogenid, wie beispielsweise Methylenchlorid, Chloroform oder Dichlorethan.

7. Verfahren nach einem der Ansprüche 1 bis 4, **dadurch gekennzeichnet, daß** die Taxane der allgemeinen Formel 3c, wie in Anspruch 4 definiert, anschließend für die Hemisynthese von Taxanen durch Veresterung des Hydroxyls in 13 mit einem geeigneten Vorläufer der Seitenkette des Taxans verwendet werden, um ein Taxan-Derivat der allgemeinen Formel 3d 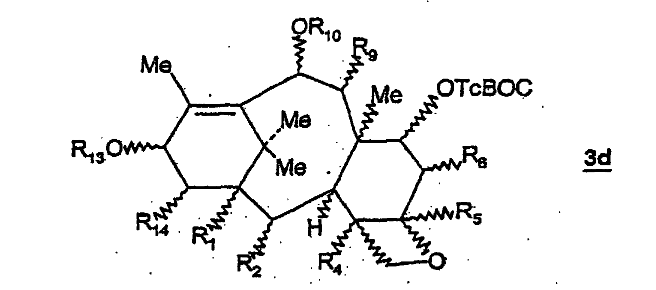 zu erhalten, in der
R₁, R₂, R₄, R₅, R₆, R₁₀ und R₁₄ wie in Anspruch 1 definiert sind, und
R₁₃ einen Rest des Vorläufers der Seitenkette des Taxans darstellt,
bei dem man anschließend die selektive Abspaltung der Schutzgruppe vom Hydroxyl in 7 durchführt, gegebenenfalls begleitet von der Öffnung und/oder der vorher erfolgten und/oder der Umwandlung und/oder der Abspaltung der Schutzgruppe vom Vorläufer der Seitenkette, um das gesuchte Taxan zu erhalten.

8. Verfahren nach Anspruch 7, **dadurch gekennzeichnet, daß** R₁₃ der Vorläufer der Seitenkette von PACLITAXEL ist.

9. Verfahren nach einem der Ansprüche 7 oder 8, **dadurch gekennzeichnet, daß** die Abspaltung der Schutzgruppe vom Hydroxyl in 7 durch ß-Eliminierung erfolgt.

10. 7-(2,2,2-Trichlor-tert.-butoxycarbonyl)-10-hydroxy-taxan-Derivate der folgenden Formel 3b: 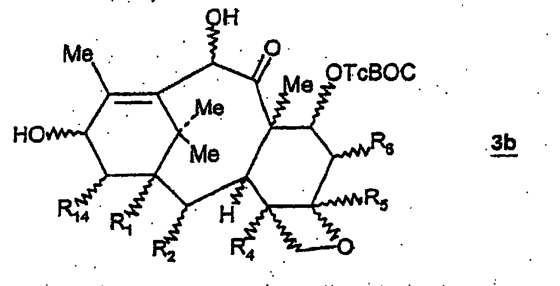 in der
R₁, R₂, R₄, R₅, R₆ und R₁₄ wie in Anspruch 1 definiert sind.

11. Derivate der folgenden Formel 3c: 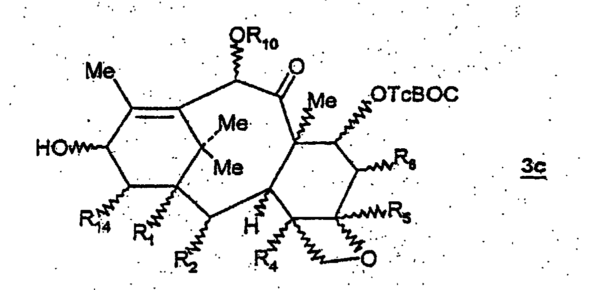 in der
R₁, R₂, R₄, R₅, R₆ und R₁₄ wie in einem der vorstehenden Ansprüche und R₁₀ wie in Anspruch 4 definiert sind.

## Claims

1. Process for preparing 7-(2,2,2-trichloro-tert-butoxycarbonyl)-10-hydroxytaxane derivatives corresponding to formula **3b** below: 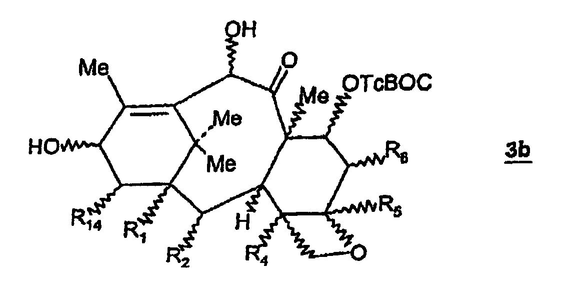 in which
R₁, R₂, R₄, R₅, R₆ and R₁₄ represent, independently of each other, a radical Q, with:
Q = H, OH, R, OR, SH, SR, OCOR, OCOOR, HCO, X, and
X = halogen,
R represents, independently of each other, a linear or branched alkyl or alkylene radical, a perhaloalkyl radical, a linear or branched heteroalkyl or heteroalkylene radical, a cycloalkyl or cycloalkylene radical, a heterocycloalkyl or heterocycloalkylene radical, or an aryl or aralkyl radical, the radicals possibly being substituted, in particular with one or more halogens,
by slow addition at a temperature above room temperature, preferably between 20 and 80°C, of 2,2,2-trichloro-tert-butoxycarbonyl chloride diluted in a suitable solvent, to a vigorously stirred solution of the 7,10-dihydroxytaxane of general formula **3a** 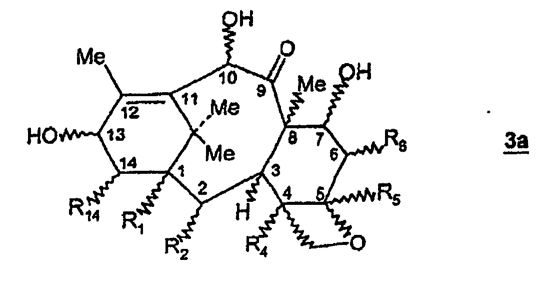 in which
R₁, R₂, R₄, R₅, R₆ and R₁₄ are defined above,
in the presence of pyridine and/or a hindered substituted pyridine such as, for example, 4-pyrrolidinopyridine or dimethylaminopyridine.

2. Process according to Claim 1, **characterized in that** the 10-hydroxytaxane of formula **3a** is the 10-deacetylbaccatin of general formula **2**

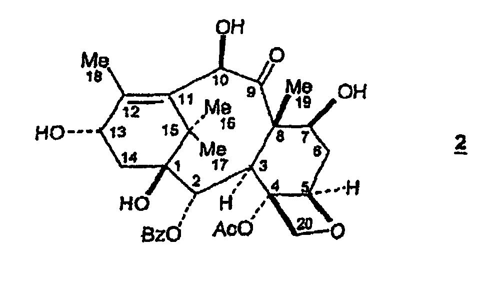

3. Process according to either of Claims 1 and 2, **characterized in that** the esterification is performed with 1 to 1.5 equivalents of 2,2,2-trichloro-tert-butoxycarbonyl chloride relative to the 7,10-dihydroxytaxane of general formula **3a**.

4. Process according to one of Claims 1 to 3, **characterized in that** a 7-(2,2,2-trichloro-tert-butoxycarbonyl)-10-acyloxytaxane derivative of general formula **3c** below: 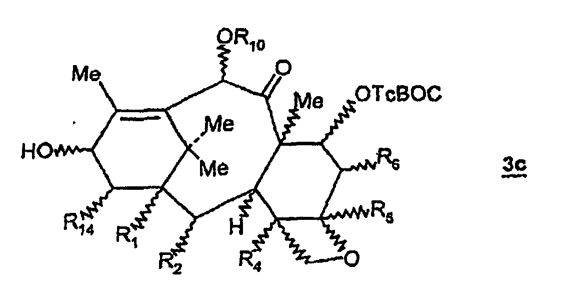 in which
R₁, R₂, R₄, R₅, R₆ and R₁₄ are defined in Claim 1, and
R₁₀ represents an acyl radical of formula O-CO-R, R being defined in Claim 1,
is then prepared by slow addition at room temperature of the corresponding acyl chloride diluted in a suitable solvent to a vigorously stirred solution of the 7-(2,2,2-trichloro-tert-butoxycarbonyl)-10-hydroxytaxane derivative of formula **3b** defined in Claim 1, in the presence of pyridine and/or of a hindered substituted pyridine such as, for example, 4-pyrrolidinopyridine or dimethylaminopyridine.

5. Process according to Claim 4, **characterized in that** R represents an alkyl group, in particular methyl.

6. Process according to one of Claims 1 to 4, **characterized in that** the suitable solvent is a non-hydroxylated solvent, in particular an alkyl halide such as, for example, methylene chloride, chloroform or dichloroethane.

7. Process according to one of Claims 1 to 4, **characterized in that** the taxanes of general formula **3c** defined in Claim 4 are then used for the hemisynthesis of taxanes, by esterification of the hydroxyl at 13 with a suitable taxane side chain precursor, to give a taxane derivative of general formula **3d** 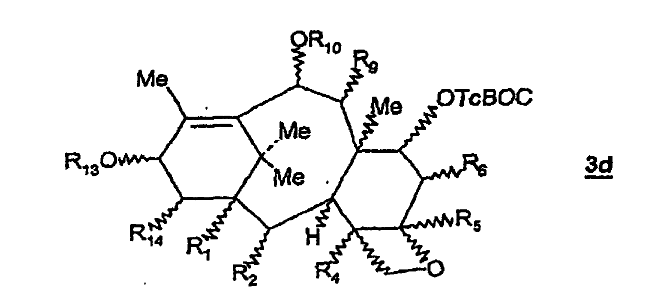 in which
R₁, R₂, R₄, R₅, R₆ and R₁₄ are defined in Claim 1, and
R₁₃ represents a radical that is a precursor of a taxane side chain,
and then by selective deprotection of the hydroxyl at 7, optionally accompanied and/or preceded by the opening and/or conversion and/or deprotection of the side chain precursor to give the desired taxane.

8. Process according to Claim 7, **characterized in that** R₁₃ is the precursor for the Paclitaxel side chain.

9. Process according to either of Claims 7 and 8, **characterized in that** the deprotection of the hydroxyl at 7 is carried out by β-elimination.

10. 7-(2,2,2-Trichloro-tert-butoxycarbonyl)-10-hydroxytaxane derivatives corresponding to formula **3b** below: 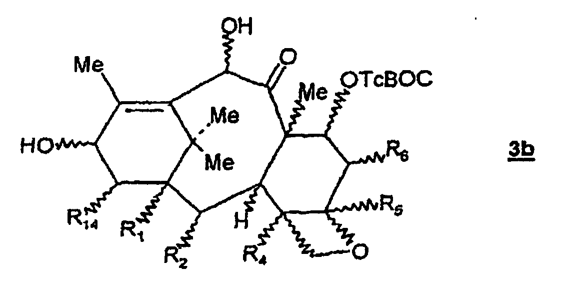 in which
R₁, R₂, R₄, R₅, R₆ and R₁₄ are defined in Claim 1.

11. Derivatives of general formula **3c** below: 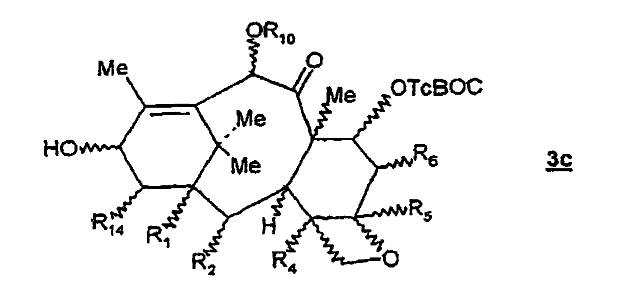 in which
R₁, R₂, R₄, R₅, R₆ and R₁₄ are defined in one of the preceding claims, and R₁₀ is defined in Claim 4.
